# EUROPÄISCHE PATENTSCHRIFT

(11) **EP 3 243 069 B1**
(45) Veröffentlichungstag und Bekanntmachung des Hinweises auf die Patenterteilung: **31.07.2019**
(21) Anmeldenummer: 16706343.7
(22) Anmeldetag: 23.02.2016
(51) Int. Cl.: G01N 21/39, G01N 33/00

(54) **LASER-BASIERTE IR-SPEKTROSKOPIE FÜR DIE MESSUNG VON SCHWEFELTRIOXID IM ABGAS VON GASKRAFTWERKEN**
LASER-BASED IR SPECTROSCOPY FOR MEASURING SULFUR TRIOXIDE IN THE EXHAUST GAS OF GAS POWER PLANTS
SPECTROSCOPIE IR À BASE DE LASER POUR LA MESURE DU TRIOXYDE DE SOUFRE DANS LE GAZ D'ÉCHAPPEMENT DES CENTRALES ÉLECTRIQUES À GAZ

(30) Priorität: 18.03.2015 DE 102015204883
(43) Veröffentlichungstag der Anmeldung: 15.11.2017
(73) Patentinhaber: Siemens Aktiengesellschaft, 80333 München (DE)
(72) Erfinder: BRECHTEL, Kevin, 91486 Uehlfeld (DE); SIGLING, Ralf, 91083 Baiersdorf (DE); RAAKE, Katrin, 63322 Rödermark (DE); SCHRAMM, Henning, 65719 Hofheim am Taunus (DE); STRZODA, Rainer, 81825 München (DE)
(86) Internationale Anmeldenummer: PCT/EP2016/053773
(87) Internationale Veröffentlichungsnummer: WO 2016/146351

(56) Entgegenhaltungen:
- US-B1- 8 368 896
- RAWLINS W T ET AL: "QUANTUM CASCADE LASER SENSOR FOR SO2 AND SO3 FOR APPLICATION TO COMBUSTOR EXHAUST STREAMS", APPLIED OPTICS, OPTICAL SOCIETY OF AMERICA, WASHINGTON, DC; US, Bd. 44, Nr. 31, 1. November 2005 (2005-11-01), Seiten 6635-6643, XP001236488, ISSN: 0003-6935, DOI: 10.1364/AO.44.006635
- R F MAJKOWSKI ET AL: "Infrared absorption coefficients of gaseous H(2)SO(4) and SO(3)", APPLIED OPTICS, Bd. 17, Nr. 7, 1. April 1978 (1978-04-01), Seiten 975-977, XP055269286, WASHINGTON, DC; US ISSN: 0003-6935, DOI: 10.1364/ao.17.000975

## Beschreibung

Die Erfindung betrifft ein Verfahren zur Bestimmung eines Schwefeltrioxid-Gehalts in einem Gas sowie ein Verfahren zum Betreiben einer Kraftwerksanlage mit einer Gasturbine und mit einem Abhitzedampferzeuger. Die Erfindung betrifft ferner ein Messsystem zur Bestimmung eines Schwefeltrioxid-Gehalts in einem Gas, sowie eine Kraftwerksanlage mit einer Gasturbine einem Abhitzedampferzeuger.

Normalerweise enthält Erdgas lediglich sehr geringe Mengen an Schwefel (S). Man geht davon aus, dass der Schwefelanteil im Brenngas für eine Gasturbine beim Verbrennungsprozess mit Sauerstoff (O₂) vollständig zu Schwefeldioxid (SO₂) umgesetzt wird.

S + O₂ → SO₂

Im Gasstrom des Abhitzedampferzeugers wird Schwefeldioxid zum Teil weiter zu Schwefeltrioxid (SO₃) umgesetzt:

SO₂ + 1/2O₂ → SO₃.

Die entstehenden Schwefeldioxid- und Schwefeltrioxid-Konzentrationen im Abgas sind jedenfalls zuerst einmal vergleichsweise klein. Trotzdem sollte die Schwefeltrioxid-Konzentration im Hinblick auf mögliche Schwefelsäurebildung und deren negative Auswirkungen auf das kalte Ende eines Abhitzedampferzeugers einer Gas-und-Dampfturbinen-Anlage bestimmt oder zumindest abgeschätzt werden, da Schwefeltrioxid mit Wasser (H₂O) zu Schwefelsäure (H₂SO₄) reagiert:

SO₃ + H₂O → H₂SO₄

Die Schwefelsäure kondensiert bei Unterschreiten des Schwefelsäuretaupunkts und führt zu Korrosion. Eine Bestimmung oder Schätzung des Schwefeltrioxid-Gehalts ist aber nicht ganz einfach, da die Konversionsrate eine Funktion verschiedener Parameter ist.

Beispielsweise erhöht sich bei Einheiten mit Katalysatoren für die selektive katalytische Reaktion oder mit Katalysatoren für Kohlenmonoxid die Schwefeldioxidumsetzung an diesen Katalysatoren; die Schwefeltrioxid-Konzentration wird also erhöht.

Ein weiterer zu berücksichtigender Faktor ist die Verweilzeit des Abgases im Abhitzedampferzeuger, welche in Abhängigkeit einer Gasturbinenlast schwankt. Je niedriger die Gasturbinenlast ist, desto länger ist auch die Verweilzeit des Abgases im Abhitzedampferzeuger und folglich steigt auch die Konversion von Schwefeldioxid zu Schwefeltrioxid.

Eine Maßnahme zur Vermeidung von Schwefelsäure ist es, sicherzustellen, dass die Temperatur des Kondensatvorwärmers im Abhitzedampferzeuger dauerhaft über dem Schwefelsäuretaupunkt liegt.

Im Interesse eines möglichst hohen Anlagen-Wirkungsgrads sollte die Temperatur des Kondensatvorwärmers jedoch so niedrig wie möglich sein.

Es ist somit gang und gäbe für Gas-und-Dampfturbinen-Anlagen eine Schätzung der Schwefeldioxid-Konversionsrate durchzuführen, welche im Hinblick auf Korrosionsprobleme eher konservativ sein wird und zu Lasten des Wirkungsgrades geht. Hierzu wird die minimale Temperatur des Rauchgases im Abhitzedampferzeuger eines Gas-und-Dampfturbinen-Kraftwerkes durch entsprechendes Design des Abhitzedampferzeugers und einer entsprechend hohen Kondensatvorlauftemperatur so hoch gewählt, dass genügend Sicherheit vorhanden ist, um den Säuretaupunkt der Schwefelsäure auf jeden Fall nicht zu unterschreiten (z.B. 10 K über dem erwarteten Taupunkt). Durch den "Sicherheitsabstand" wird das Abgas im Abhitzedampferzeuger nicht soweit gekühlt, wie es theoretisch ohne Auftreten von Schwefelsäurekorrosion möglich wäre. Hierdurch wird ggf. Wärme im Bereich von mehreren MW "verschenkt", der Wirkungsgrad des Gas-und-Dampfturbinen-Kraftwerks sinkt. Es wäre daher wünschenswert, den Schwefeltrioxid-Gehalt im Abgas genauer bestimmen zu können.

Der Schwefeltrioxid-Gehalt bzw. Schwefelsäure-Gehalt des Abgases kann durch Probenentnahme und chemische Analyse im Labor erfolgen. Ein solches Verfahren ist aber langsam, aufwändig und kostspielig [Continuous Measurement Technologies for SO₃ and H₂SO₄ in Coal-Fired Power Plants, EPRI, Palo Alto, CA: 2004. 1009812.].

Der Schwefelanteil in Kohle führt bei Kohlekraftwerken zu Schwefeltrioxid-Konzentrationen im Abgas vom 1 bis 100 ppm. Für diesen Konzentrationsbereich gibt es Sonden, die den Schwefelsäuregehalt in-situ nach dem Verfahren der kontrollierten Kondensation bestimmen [Continuous Measurement Technologies for SO₃ and H₂SO₄ in Coal-Fired Power Plants, EPRI, Palo Alto, CA: 2004. 1009812.].

Für den gleichen Konzentrationsbereich gibt auch einen Ansatz in-situ mit mittels Laserspektroskopie direkt im Abgas zu messen [http://practices.geosyntec.com/airquality/pdf/Geosyntec-SO3-Analyzer-Technical-Brief.pdf]. Extraktive Verfahren mit Laserspektroskopie sind auch bekannt. Die Verfahren sind jedoch bisher auf vergleichsweise hohe Schwefeltrioxid-Konzentrationen (0,5 ppm - 200 ppm) beschränkt [http://www.psicorp.com/pdf/library/sr-1210.pdf], [http://www.ayt.cl/files/articulos/WP_AQISO3_0810.pdf], [US 8,368,896 B1].

Die Schwefeltrioxid-Konzentrationen, die man im Abgas eines Gas-betriebenen Kraftwerkes erwartet, liegen im 10 bis 1000 ppb-Bereich.

Aufgabe der Erfindung ist es daher, ein Verfahren zur Bestimmung eines Schwefeltrioxid-Gehalts in einem Gas, insbesondere in einem Abgas einer Gasturbine anzugeben, damit die Temperatur am kalten Ende eines Abhitzedampferzeugers möglichst niedrig gehalten werden kann, um den Wirkungsgrad einer Kraftwerksanlage zu steigern. Eine weitere Aufgabe der Erfindung ist die Angabe eines Verfahrens zum Betreiben einer Kraftwerksanlage mit einer Gasturbine und mit einem Abhitzedampferzeuger. Aufgabe der Erfindung ist es ferner, ein entsprechendes Messsystem zur Bestimmung eines Schwefeltrioxid-Gehalts in einem Gas bzw. eine entsprechende Kraftwerksanlage anzugeben.

Die Erfindung löst die auf ein Verfahren zur Bestimmung eines Schwefeltrioxid-Gehalts in einem Gas gerichtete Aufgabe, indem sie vorsieht, dass bei einem derartigen Verfahren, bei dem eine Probe des Gases entnommen und ein Gasdruck der Probe verringert wird, und mit einer Wellenzahl-abstimmbaren monochromen Lichtquelle an der Probe eine Wellenzahl-aufgelöste Transmissionsmessung durchgeführt wird und aus der Messung ein Schwefeltrioxid-Gehalt abgeleitet wird, die Messung in der Schwefeltrioxid-Absorptionsbande zwischen 1360 und 1410 cm⁻¹, in einem Fenster um die Schwefeltrioxid-Absorption bei 1365,49 cm⁻¹, unter Nutzung dieser Schwefeltrioxid-Absorption erfolgt.

Angewandt wird die Spektroskopie typischerweise mit abstimmbaren Halbleiterlasern. Mit der abstimmbaren Lichtquelle wird eine Wellenzahl-aufgelöste Transmissionsmessung durchgeführt. Das transmittierte Licht wird von einem Fotodetektor aufgefangen.

Die spektrale Messung erfolgt in einem extraktiven System, d.h. dem Gas wird eine Probe entnommen und einer Messzelle zugeführt. In der Messzelle wird das Gas auf einem reduzierten Druck gehalten, um die Linienbreite der Absorptionslinien zu verkleinern und damit überhaupt erst die Trennung der Spektren der einzelnen Gase zu ermöglichen. Bei Normaldruck überlagert die Wasser-Absorption den größten Teil der Schwefeltrioxid-Bande und verhindert so eine selektive Messung der Schwefeltrioxid-Konzentration im niedrigen Konzentrationsbereich.

Die Nutzung der Schwefeltrioxid-Absorption bei 1365,49 cm⁻¹ in Verbindung mit einem reduzierten Druck in der Absorptionsmesszelle bietet eine Reihe von Vorteilen. Die Messung erfolgt in einer spektralen Lücke des Wasserspektrums, d.h. der Spektralbereich ist weitgehend transparent. In der Lücke treten die Absorption des Zielgases und weiterer Abgaskomponenten auf.

Die Absorption von Schwefeltrioxid im Zielwellenzahlfenster weist im Vergleich zu den Nachbarlinien eine um mindestens den Faktor 2 größere Amplitude und eine schlankere Form auf als die der benachbarten Schwefeltrioxid-Absorptionen. Die größere Absorption erleichtert die Realisierung einer niedrigen Nachweisgrenze, die schmale Linie erleichtert die Selektion gegenüber den potentiell interferierenden Absorptionen der anderen Abgaskomponenten. Die übrigen Abgaskomponenten im vorkommenden Konzentrationsbereich führen nur zu einer geringen Störung des Schwefeltrioxid-Spektrums.

Um die Linienbreite der Absorptionslinien zu verkleinern und damit überhaupt erst die Trennung der Spektren der einzelnen Gase zu ermöglichen, ist es erforderlich, das Gas in der Messzelle auf einem reduzierten Druck zu halten. Insbesondere ist es vorteilhaft, wenn die Probe auf einen Druck unter 100 hPa gebracht wird.

Im Hinblick auf die durch die Boltzmann-Verteilung und die Zustandsdichte bestimmte Linienintensität und somit auch im Hinblick auf die Nachweisgrenze ist es im Fall der Schwefeltrioxid-Absorption bei 1365,49 cm-1 vorteilhaft, wenn eine Probentemperatur über dem Taupunkt von Schwefelsäure, beispielsweise bei 200°C, eingestellt wird.

Es ist weiterhin vorteilhaft, wenn die Messung mittels Wellenlängenmodulationsspektroskopie (WMS) mit Detektion der 2. Harmonischen (oder auch höhere Harmonische) erfolgt, weil diese Technik den Einfluss von Nachbarlinien auf das Spektrum des Zielgases minimiert. Insbesondere tritt eine teilweise Überlagerung der Absorptionslinie von Schwefeltrioxid mit Schwefeldioxid tritt. Die Anwendung der Wellenlängenmodulationsspektroskopie bei optimaler Einstellung der Modulationsamplitude unterdrückt die Schwefeldioxid-Absorption zugunsten der Schwefeltrioxid-Linie.

Dabei ist es zweckmäßig, wenn die spektrale Messung in einer Langwegzelle mit einem mehrfach gefalteten Strahlengang durchgeführt wird, insbesondere wenn eine Absorptionsstrecke maximal 15 m, vorzugsweise maximal 10 m, insbesondere maximal 5 m beträgt. Damit erreicht man mit der Ziel-Absorptionslinie bei 1365,49 cm⁻¹ eine Absorption bei 1 ppm im 1 %-Bereich.

Es ist weiterhin zweckmäßig, wenn ein gemessenes Spektrum in einer Kurvenanpassung mit einem Modellspektrum verglichen wird und die Konzentration von Schwefeltrioxid als Parameter in das Modellspektrum eingeht.

Ferner ist es zweckmäßig, wenn neben der Konzentration von Schwefeltrioxid auch Konzentrationen interferierender Gase in das Modell entweder als bekannte Werte, die aus einer zweiten unabhängigen Messmethode hervorgehen, oder als Fitparameter eingehen.

Die auf ein Verfahren zum Betreiben einer Kraftwerksanlage mit einer Gasturbine und einem Abhitzedampferzeuger gerichtete Aufgabe wird dadurch gelöst, dass ein Schwefeltrioxid-Gehalt im Abgas der Gasturbine bestimmt wird und eine Abgastemperatur im Abhitzedampferzeuger auf Grundlage des Schwefeltrioxid-Gehalts so angepasst wird, dass ein Schwefelsäuretaupunkt im Abhitzedampferzeuger nicht unterschritten wird.

Die auf ein Messsystem zur Bestimmung eines Schwefeltrioxid-Gehalts in einem Gas gerichtete Aufgabe wird gelöst durch ein Messsystem zur Bestimmung eines Schwefeltrioxid-Gehalts in einem Gas, umfassend eine erste Gasleitung von einer Gasentnahmestelle zu einer Messzelle, eine Druckregeleinrichtung für die Messzelle, eine Wellenzahl-abstimmbare monochrome Lichtquelle im Bereich einer Schwefeltrioxid-Absorptionsbande, eine Steuerung für die Durchführung einer Transmissionsmessung in der Messzelle, sowie eine Auswerteeinheit zur Bestimmung des Schwefeltrioxid-Gehalts, wobei die Lichtquelle geeignet ist, monochromatisches Licht zwischen 1360 und 1410 cm⁻¹, in einem Fenster um die Schwefeltrioxid-Absorption bei 1365,49 cm⁻¹, zu erzeugen.

In einer vorteilhaften Ausführungsform der Erfindung umfasst die Druckregeleinrichtung einen Druckregler und eine Vakuumpumpe, welche über eine zweite Gasleitung mit der Messzelle verbunden sind.

In einer weiteren vorteilhaften Ausführungsform ist mindestens eines von beiden, erste Gasleitung und Messzelle, beheizbar. Somit kann bei der gewählten Wellenzahl die Linienintensität gegenüber Raumtemperatur bzw. der Temperatur an der Abzweigstelle gesteigert und die Nachweisgenauigkeit von Schwefeltrioxid verbessert werden.

Um Partikel vom Messsystem fernzuhalten, ist es vorteilhaft, wenn das Messsystem einen in die erste Gasleitung geschalteten Partikelfilter umfasst.

Zweckmäßigerweise umfasst das Messsystem eine in die erste Gasleitung geschaltete Drosseleinrichtung. Die Drosseleinrichtung folgt in Strömungsrichtung des Gases auf das Filter. Die Drosseleinrichtung kann eine einfache Drossel sein oder alternativ ein Massendurchflussregler, der den gewünschten Durchsatz bei Unterdruck-Betrieb bereitstellt.

In einer vorteilhaften Ausführungsform der Erfindung ist die Messzelle eine Langwegzelle mit einem mehrfach gefalteten Strahlengang, der eine Absorptionsstrecke von 5 bis 15 m, vorzugsweise 7 bis 12 m aufweist.

Gemäß der Erfindung umfasst eine Kraftwerksanlage mit einer Gasturbine und einem Abhitzedampferzeuger auch ein solches Messsystem.

Im Gegensatz zum in der US 8,368,896 B1 offenbarten Verfahren, welches die Absorption von Schwefeltrioxid bei 1397 cm⁻¹ nutzt, die auch bei Schwefeldioxid-Konzentrationen > 100 ppm weitgehend unbeeinflusst von der Schefeldioxid-Konzentration ist, weist die Absorption bei 1365,49 cm⁻¹ des erfindungsgemäßen Verfahrens eine teilweise Überlagerung mit der Schwefeldioxid-Absorption auf. D. h. es müssen beide Gaskomponenten in der Auswertung berücksichtigt werden, um den Quereinfluss zu kompensieren. Das bedeutet aber auch, dass man beide Komponenten in einer einzigen spektralen Messung erfassen und quantitativ auswerten kann. Die Aufgabe wird im Zielwellenzahlfenster dadurch erleichtert, dass eine weitere Schwefeldioxid-Absorption getrennt von der Schwefeltrioxid-Absorption auftritt (zwischen 1365,52 und 1365,54 cm⁻¹) und damit eine unabhängige Schwefeldioxid-Messung ermöglicht wird, was die Auswertung erleichtert.

Wegen der Stärke der Schwefeldioxid-Absorption im Verhältnis zur Schwefeltrioxid-Absorption eignet sich die Messmethode am besten für Erdgas-befeuerte Kraftwerke bei denen nur Schwefeldioxid-Konzentrationen < 5 ppm auftreten. Die Messmethode ist auch anwendbar für Öl-befeuerte Kraftwerke, bei denen Schwefeldioxid-Konzentrationen < 50 ppm auftreten. Für Kohlebefeuerte Kraftwerke mit Schwefeldioxid-Konzentrationen > 100 ppm wird die Genauigkeit der Schwefeltrioxid-Messung abnehmen, weil die Korrektur der Schwefeltrioxid-Absorption mit der Schwefeldioxid-Absorption schwieriger wird.

Von der Absorptionsstärke her ist die Absorption von Schwefeltrioxid bei 1365,49 cm⁻¹ der Absorption von Schwefeltrioxid bei 1397 cm⁻¹ überlegen, was für die Auswahl der 1365,49 cm⁻¹-Absorption für Anwendungen mit einer Nachweisgrenze im ppb-Bereich spricht. Die US 8,368,896 B1 offenbart eine Absorption der Linien bei einer Messzellenlänge von 12 m und 1 ppm Schwefeltrioxid bei 443 K im unteren Promille-Bereich. Die Simulation der Absorptionsstärke mit den Daten der HITRAN-Datenbasis (Abkürzung für "**Hi**gh Resolution **Tran**smission", eine Sammlung spektroskopischer Parameter für die Simulation von Transmission und Emission, unterhalten vom Harvard-Smithsonian Center for Astrophysics, Cambridge MA, USA) ergibt für Raumtemperatur eine Absorptionsstärke um 1 %. Die Daten der HITRAN-Datenbasis erlauben für Schwefeltrioxid nicht die Berechnung der Spektren bei erhöhter Temperatur. Eine Abschätzung mit den vorhandenen Daten ergibt bei 200°C eine Steigerung der Absorption um einen Faktor 2 bis 3. D.h. die Absorptionsstärke zwischen der Absorption bei 1365,49 cm⁻¹ und dem Bereich um 1397 cm⁻¹ unterscheidet sich um eine Größenordnung, was sich in der erreichbaren Nachweisempfindlichkeit auswirkt.

Im Gegensatz zu bisherigen Berechnungen/Schätzungen des Schwefeltrioxid-Anteiles im Rauchgas und dem entsprechendem Design/Betrieb des Abhitzedampferzeugers mit erheblichen Sicherheitszuschlägen wird die Schwefeltrioxid-Konzentration im Rauchgas jetzt direkt gemessen und für die Einstellung/Regelung der Kondensatvorlauftemperatur genutzt.

Durch den Einsatz der Messung für eine Regelung der Kondensatvorlauftemperatur kann das Rauchgas im Abhitzedampferzeuger nun wesentlich weiter gekühlt werden, wodurch mehr Dampf erzeugt werden kann. Der Wirkungsgrad des Gas-und-Dampfturbinen-Kraftwerkes steigt. Außerdem ist durch eine Regelung der Kondensatvorlauftemperatur sichergestellt, dass die Temperatur des Abgases immer über dem Säuretaupunkt liegt und Säurekorrosion so nicht auftreten kann. Dies bedeutet eine erhöhte Sicherheit gegenüber dem bisherigen Vorgehen (da Sicherheitszuschläge bei sehr ungenauer Schätzung der Schwefeltrioxid-Konzentration keine Garantie bieten, dass der Zuschlag auch hoch genug war - z.B. bei zeitlichen Schwankungen des Schwefelanteils im Brenngas der Gasturbine, wobei dieser dem Betreiber im Allg. nur als Monatsmittelwert garantiert wird.

Eine minutengenaue Anpassung der Abgastemperatur wäre möglich und umso effektiver (hinsichtlich Korrosionssicherheit und Kraftwerkswirkungsgrad), da in neuerer Zeit verstärkt verschiedene/schwankende Gasqualitäten (Russland, Norwegen, Nordsee, USA, ...) zum Einsatz kommen.

Die Flexibilität des Kraftwerkes erhöht sich, da man mit entsprechendem Wissen über die Schwefeltrioxid-Konzentration im Abgas (besonders bei Teillast ist die Umsetzungsrate SO₂→SO₃ schwer ohne Messung vorhersagbar) mit entsprechenden Maßnahmen in die Steuerung des Kraftwerkes eingreifen kann.

Die Erfindung wird beispielhaft anhand der Zeichnungen näher erläutert. Es zeigen schematisch und nicht maßstäblich:
- Figur 1: eine Gas- und Dampfturbinenanlage,
- Figur 2: ein Messsystem nach der Erfindung,
- Figur 3: das Verfahren zur Bestimmung eines Schwefeltrioxid-Gehalts in einem Gas nach der Erfindung und
- Figur 4: ein WMS-Spektrum (2. Harmonische) von Schwefeltrioxid und Schwefeldioxid um 1365,5 cm^{-1.}

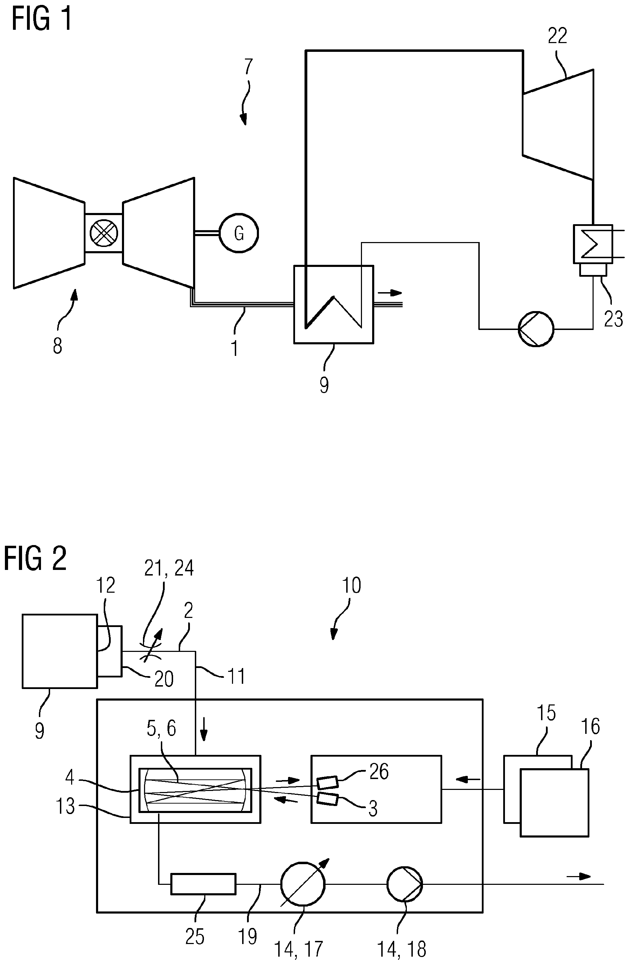

Die Figur 1 zeigt schematisch und beispielhaft eine Kraftwerksanlage 7, insbesondere eine Gas- und Dampfturbinenanlage. Mit dem Abgas 1 der Gasturbine 8 wird im der Gasturbine 8 nachgeschalteten Abhitzedampferzeuger 9 Dampf für den Betrieb der Dampfturbine 22 erzeugt. In der Dampfturbine 22 entspannter Dampf wird im Kondensator 23 kondensiert und dem Abhitzedampferzeuger 9 erneut zugeführt.

Figur 2 zeigt ein Messsystem 10 nach der Erfindung. Die Gasentnahme erfolgt am Abhitzedampferzeuger 9 an einer geeigneten Gasentnahmestelle 12 (an der Stelle, an der die niedrigste Temperatur zu erwarten ist). Um Partikel vom Messsystem 10 fernzuhalten, passiert die Probe 2 (d.h. das Messgas) zunächst ein Partikelfilter 20 mit angepasster Porengröße, das den Betriebsbedingungen am Einsatzort standhält. Eine Möglichkeit wäre ein PTFE-Filter, das bis 260 °C temperaturstabil ist und mit verschiedenen Porengrößen geliefert wird. Auf das Filter 20 folgt eine Drosseleinrichtung 21, beispielsweise eine einfache Drossel oder alternativ ein Massendurchflussregler 24, der den gewünschten Durchsatz bei Unterdruck-Betrieb bereitstellt. Eine erste Gasleitung 11 von der Gasentnahmestelle 12 zur Messzelle 13 sollte wegen der thermischen Ausdehnung des Abhitzedampferzeugers 9 flexibel, auf 200°C beheizbar und möglichst kurz ausgeführt werden, um die Verweilzeit der Probe 2 gering zu halten. Je größer das Verhältnis von Oberfläche zu Volumen der ersten Gasleitung 11 ist, desto größer ist die Gefahr, dass Wechselwirkung zwischen Probe 2 und Oberfläche wie Adsorption oder katalytische Umwandlungsprozesse in störendem Umfang stattfinden. Inerte Oberflächen reduzieren die Wechselwirkungen weiter. Teflonschläuche sind bewährt für den Transport von Messgasen, wenn Konzentrationen im sub-ppm Bereich zu messen sind. Alternativ kann man Edelstahloberflächen mit einer Siliziumdioxid-Beschichtung versehen lassen, was die Wechselwirkung mit der Oberfläche reduziert. Da die Spektroskopie eine Trocknung der Probe 2 nicht erfordert, wird auf eine Gaskonditionierung verzichtet. Damit ist eine weitere Quelle für eine Verfälschung der Gaszusammensetzung eliminiert. Aus der geheizten ersten Gasleitung 11 wird die Probe 2 in die ebenso geheizte Messzelle 13 geleitet. Die benötigte Absorptionsstrecke 6 (etwa 10 m) wird von einer Langwegzelle 4 für einen mehrfach gefalteten Strahlengang 5 bereitgestellt. Nach der Messzelle 13 wird die Probe 2 in einem Gaskühler 25, der in eine zweite Gasleitung 19 geschaltet ist, auf Temperaturen abgekühlt, die für den Druckregler 17 einer Druckregeleinrichtung 14 verträglich sind. Der Druckregler 17 hält den Druck in der Messzelle 13 konstant auf Niederdruck (z.B. 100 hPa), der Druck bei dem die spektralen Linienbreiten so gering sind, dass sich die einzelnen Absorptionslinien trennen lassen. Eine Vakuumpumpe 18 erzeugt den für den Betrieb des Druckreglers 17 nötigen Unterdruck. Für die Untersuchung der Probe 2 in der Messzelle 13 werden eine Wellenzahl-abstimmbare monochrome Lichtquelle 3 (Laser), eine Photodiode 26, sowie ferner eine Steuerung 15 für die Durchführung einer Transmissionsmessung und eine Auswerteeinheit 16 benötigt.

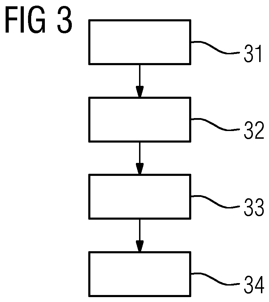

Figur 3 zeigt das Verfahren zur Bestimmung eines Schwefeltrioxid-Gehalts in einem Gas, bei dem in einem ersten Schritt 31 eine Probe 2 des Gases 1 entnommen und in einem zweiten Schritt 32 ein Gasdruck der Probe 2 verringert wird, und in einem dritten Schritt 33 mit einer Wellenzahl-abstimmbaren monochromen Lichtquelle 3 an der Probe 2 eine Wellenzahl-aufgelöste Transmissionsmessung in der Schwefeltrioxid-Absorptionsbande zwischen 1360 und 1410 cm⁻¹, in einem Fenster um die Schwefeltrioxid-Absorption bei 1365,49 cm⁻¹ durchgeführt wird und in einem vierten Schritt 34 aus der Messung ein Schwefeltrioxid-Gehalt abgeleitet wird.

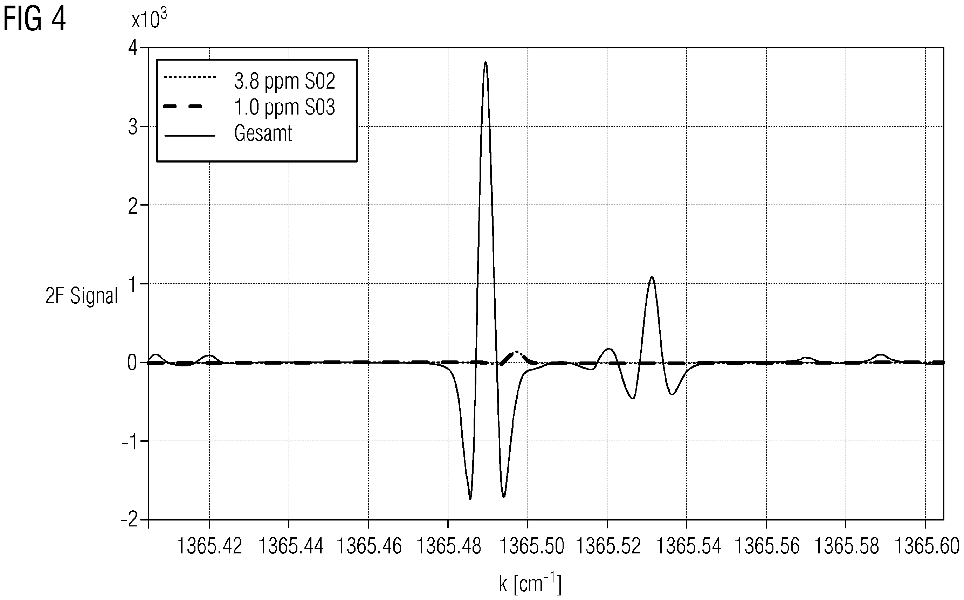

Die Figur 4 zeigt ein Wellenlängenmodulationsspektrum (2. Harmonische) von Schwefeltrioxid und Schwefeldioxid um 1365,5 cm⁻¹. Das Wellenlängenmodulationsspektroskopie-Verfahren eliminiert den Einfluss der Wasserabsorption im Bereich der Schwefeltrioxid Absorption praktisch vollständig.

## Patentansprüche

1. Verfahren zur Bestimmung eines Schwefeltrioxid-Gehalts in einem Gas (1), bei dem eine Probe (2) des Gases (1) entnommen und ein Gasdruck der Probe (2) verringert wird, und mit einer Wellenzahl-abstimmbaren monochromen Lichtquelle (3) an der Probe (2) eine Wellenzahl-aufgelöste Transmissionsmessung durchgeführt wird und aus der Messung ein Schwefeltrioxid-Gehalt abgeleitet wird, **dadurch gekennzeichnet, dass** die Messung in der Schwefeltrioxid-Absorptionsbande zwischen 1360 und 1410 cm⁻¹ erfolgt, **dadurch gekennzeichnet, dass** die Messung in einem Fenster um die Schwefeltrioxid-Absorption bei 1365,49 cm⁻¹, unter Nutzung dieser Schwefeltrioxid-Absorption erfolgt.

2. Verfahren nach Anspruch 1, wobei die Probe (2) auf einen Druck unter 100 hPa gebracht wird.

3. Verfahren nach einem der Ansprüche 1 oder 2, wobei eine Probentemperatur über 200°C eingestellt wird.

4. Verfahren nach einem der vorhergehenden Ansprüche, wobei die Messung mittels Wellenlängenmodulationsspektroskopie (WMS) mit Detektion der 2. Harmonischen (oder auch höhere Harmonische) erfolgt.

5. Verfahren nach einem der vorhergehenden Ansprüche, wobei die spektrale Messung in einer Langwegzelle (4) mit einem mehrfach gefalteten Strahlengang (5) durchgeführt wird.

6. Verfahren nach Anspruch 5, wobei eine Absorptionsstrecke (6) maximal 15 m, vorzugsweise maximal 10 m, insbesondere maximal 5 m beträgt.

7. Verfahren nach einem der vorhergehenden Ansprüche, wobei ein gemessenes Spektrum in einer Kurvenanpassung mit einem Modellspektrum verglichen wird und die Konzentration von Schwefeltrioxid als Parameter in das Modellspektrum eingeht.

8. Verfahren nach Anspruch 7, wobei neben der Konzentration von Schwefeltrioxid auch Konzentrationen interferierender Gase in das Modell entweder als bekannte Werte, die aus einer zweiten unabhängigen Messmethode hervorgehen, oder als Fitparameter eingehen.

9. Verfahren zum Betreiben einer Kraftwerksanlage (7) mit einer Gasturbine (8) und mit einem Abhitzedampferzeuger (9), wobei ein Schwefeltrioxid-Gehalt im Abgas (1) der Gasturbine nach einem der vorhergehenden Ansprüche bestimmt wird und eine Abgastemperatur im Abhitzedampferzeuger (9) auf Grundlage des Schwefeltrioxid-Gehalts so angepasst wird, dass ein Schwefelsäuretaupunkt im Abhitzedampferzeuger (9) nicht unterschritten wird.

10. Messsystem (10) zur Bestimmung eines Schwefeltrioxid-Gehalts in einem Gas (1), umfassend eine erste Gasleitung (11) von einer Gasentnahmestelle (12) zu einer Messzelle (13), eine Druckregeleinrichtung (14) für die Messzelle (13), eine Wellenzahl-abstimmbare monochrome Lichtquelle (3) im Bereich einer Schwefeltrioxid-Absorptionsbande, eine Steuerung (15) für die Durchführung einer Transmissionsmessung in der Messzelle (13), sowie eine Auswerteeinheit (16) zur Bestimmung des Schwefeltrioxid-Gehalts, wobei die Wellenzahl-abstimmbare monochrome Lichtquelle (3) geeignet ist, monochromatisches Licht zwischen 1360 und 1410 cm⁻¹ zu erzeugen, **dadurch gekennzeichnet, dass** die Lichtquelle (3) geeignet ist, monochromatisches Licht in einem Fenster um die Schwefeltrioxid-Absorption bei 1365,49 cm⁻¹, zu erzeugen.

11. Messsystem (10) nach Anspruch 10, wobei die Druckregeleinrichtung (14) einen Druckregler (17) und eine Vakuumpumpe (18) umfasst, welche über eine zweite Gasleitung (19) mit der Messzelle (13) verbunden sind.

12. Messsystem (10) nach einem der Ansprüche 10 oder 11, wobei mindestens eines von beiden, erste Gasleitung (11) und Messzelle (13), beheizbar ist.

13. Messsystem (10) nach einem der Ansprüche 10 bis 12, weiter umfassend einen in die erste Gasleitung (11) geschalteten Partikelfilter (20).

14. Messsystem (10) nach einem der Ansprüche 10 bis 13, weiter umfassend eine in die erste Gasleitung (11) geschaltete Drosseleinrichtung (21).

15. Messsystem (10) nach einem der Ansprüche 10 bis 14, wobei die Messzelle (13) eine Langwegzelle (4) mit einem mehrfach gefalteten Strahlengang (5) ist, der eine Absorptionsstrecke (6) von 5 bis 15 m, vorzugsweise 7 bis 12 m aufweist.

16. Kraftwerksanlage (7) mit einer Gasturbine (8), einem Abhitzedampferzeuger (9) und einem Messsystem (10) nach einem der Ansprüche 10 bis 15.

## Claims

1. Method for determining a sulfur trioxide content in a gas (1), in which a sample (2) of the gas (1) is taken and a gas pressure of the sample (2) is reduced, and with a wave number tunable monochrome light source (3) a wave number resolved transmission measurement is carried out on the sample (2) and from the measurement a sulfur trioxide content is derived, **characterized in that** the measurement takes place in the sulfur trioxide absorption band between 1360 and 1410 cm⁻¹, **characterized in that** the measurement takes place in a window around the sulfur trioxide absorption at 1365.49 cm⁻¹, using this sulfur trioxide absorption.

2. Method according to Claim 1, wherein the sample (2) is brought to a pressure below 100 hPa.

3. Method according to either of Claims 1 and 2, wherein a sample temperature above 200°C is set.

4. Method according to one of the preceding claims, wherein the measurement takes place by means of wave length modulation spectroscopy (WMS) with detection of the 2^{nd} harmonic (or else higher harmonics).

5. Method according to one of the preceding claims, wherein the spectral measurement is carried out in a long path cell (4) with a multiply folded beam path (5).

6. Method according to Claim 5, wherein an absorption section (6) is a maximum of 15 m, preferably a maximum of 10 m, in particular a maximum of 5 m.

7. Method according to one of the preceding claims, wherein a measured spectrum is compared in a curve fitting with a model spectrum, and the concentration of sulfur trioxide enters as parameter into the model spectrum.

8. Method according to Claim 7, wherein in addition to the concentration of sulfur trioxide, concentrations of interfering gases also enter into the model either as known values which originate from a second independent measuring method, or as fit parameters.

9. Method for operating a power plant (7) with a gas turbine (8) and with a heat recovery steam generator (9), wherein a sulfur trioxide content in the exhaust gas (1) of the gas turbine is determined according to one of the preceding claims and an exhaust gas temperature in the heat recovery steam generator (9) is adapted on the basis of the sulfur trioxide content so that a sulfuric acid dew point is not fallen below in the heat recovery steam generator (9).

10. Measuring system (10) for determining a sulfur trioxide content in a gas (1), comprising a first gas line (11) from a gas take-off point (12) to a measuring cell (13), a pressure regulation device (14) for the measuring cell (13), a wave number tunable monochrome light source (3) in the region of a sulfur trioxide absorption band, a control (15) for carrying out a transmission measurement in the measuring cell (13), and an evaluation unit (16) for determining the sulfur trioxide content, wherein the wave number tunable monochrome light source (3) is suited to generate monochromatic light between 1360 and 1410 cm⁻¹, **characterized in that** the light source (3) is suited to generate monochromatic light in a window around the sulfur trioxide absorption at 1365.49 cm⁻¹.

11. Measuring system (10) according to Claim 10, wherein the pressure regulation device (14) comprises a pressure regulator (17) and a vacuum pump (18), which are connected to the measuring cell (13) via a second gas line (19).

12. Measuring system (10) according to either of Claims 10 and 11, wherein at least one of the two, first gas line (11) and measuring cell (13), is heatable.

13. Measuring system (10) according to one of Claims 10 to 12, further comprising a particle filter (20) connected into the first gas line (11).

14. Measuring system (10) according to one of Claims 10 to 13, further comprising a throttle device (21) connected into the first gas line (11).

15. Measuring system (10) according to one of Claims 10 to 14, wherein the measuring cell (13) is a long path cell (4) with a multiply folded beam path (5), which has an absorption section (6) of 5 to 15 m, preferably 7 to 12 m.

16. Power plant (7) with a gas turbine (8), a heat recovery steam generator (9) and a measuring system (10) according to one of Claims 10 to 15.

## Revendications

1. Procédé de détermination de la teneur en trioxyde de soufre d'un gaz (1), dans lequel on prélève un échantillon (2) du gaz (1) et on diminue une pression de gaz de l'échantillon (2) et, par une source (3) lumineuse monochromatique pouvant être accordée en nombre d'onde, on effectue sur l'échantillon (2) une mesure de transmission résolue en nombre d'onde et on déduit de la mesure une teneur en trioxyde de soufre, **caractérisé en ce que** l'on effectue la mesure dans la bande d'absorption du trioxyde de soufre entre 1360 et 1410 cm⁻¹, **caractérisé en ce que** l'on effectue la mesure dans une fenêtre autour de l'absorption du trioxyde de soufre à 1365,49 cm⁻¹, en utilisant cette absorption du trioxyde de soufre.

2. Procédé suivant la revendication 1, dans lequel on met l'échantillon (2) sous une pression inférieure à 100 hPa.

3. Procédé suivant l'une des revendications 1 ou 2, dans lequel on met l'échantillon à une température supérieure à 200°C.

4. Procédé suivant l'une des revendications précédentes, dans lequel on effectue la mesure au moyen d'une spectroscopie en modulation de longueur d'onde (WMS) avec détection du deuxième harmonique (ou également d'harmoniques supérieures).

5. Procédé suivant l'une des revendications précédentes, dans lequel on effectue la mesure spectrale dans une cellule (4) à trajet long à marche (5) des rayons coudée plusieurs fois.

6. Procédé suivant la revendication 5, dans lequel une section (6) d'absorption est au maximum de 15 m, de préférence au maximum de 10 m, notamment au maximum de 5 m.

7. Procédé suivant l'une des revendications précédentes, dans lequel on compare un spectre mesuré suivant un ajustement de courbe à un spectre modèle et on entre la concentration de trioxyde de soufre comme paramètre dans le spectre modèle.

8. Procédé suivant la revendication 7, dans lequel, outre la concentration de trioxyde de soufre, on entre également dans le modèle des concentrations de gaz qui interfèrent, soit sous la forme de valeurs connues, qui proviennent d'une deuxième méthode de mesure indépendante, soit comme paramètre d'ajustement.

9. Procédé pour faire fonctionner une centrale (7) électrique ayant une turbine (8) à gaz et un générateur (9) de vapeur à récupération de la chaleur perdue, dans lequel on détermine la teneur en trioxyde de soufre du gaz (1) d'échappement de la turbine à gaz suivant l'une des revendications précédentes et on adapte une température du gaz d'échappement dans le générateur (9) de vapeur à récupération de la chaleur perdue sur la base de la teneur en trioxyde de soufre, de manière à ne pas passer en dessous du point de rosée de l'acide sulfurique dans le générateur (9) de vapeur à récupération de la chaleur perdue.

10. Système (10) de mesure pour déterminer la teneur en trioxyde de soufre d'un gaz (1), comprenant un premier conduit (11) pour du gaz, allant d'un point (12) de prélèvement de gaz à une cellule (13) de mesure, un dispositif (14) de réglage de la pression de la cellule (13) de mesure, une source (3) lumineuse monochromatique pouvant être accordée en nombre d'onde dans la région d'une bande d'absorption du trioxyde de soufre, une commande (15) pour effectuer une mesure de transmission dans la cellule (13) de mesure, ainsi qu'une unité (16) d'exploitation pour déterminer la teneur en trioxyde de soufre, la source (3) lumineuse monochromatique accordable en nombre d'onde étant propre à produire de la lumière monochromatique entre 1360 et 1410 cm⁻¹, **caractérisé en ce que** la source (3) lumineuse est propre à produire de la lumière monochromatique dans une fenêtre autour de l'absorption du trioxyde de soufre à 1365,49 cm⁻¹.

11. Système (10) de mesure suivant la revendication 10, dans lequel le dispositif (14) de réglage de la pression comprend un régleur (17) de pression et une pompe (18) à vide, qui communiquent avec la cellule (13) de mesure par un deuxième conduit (19) pour du gaz.

12. Système (10) de mesure suivant l'une des revendications 10 ou 11, dans lequel au moins l'un des deux, à savoir le premier conduit (11) pour du gaz et la cellule (13) de mesure, peut être chauffé.

13. Système (10) de mesure suivant l'une des revendications 10 à 12, qui comprend un filtre (20) de particules monté dans le premier conduit (11) pour du gaz.

14. Système (10) de mesure suivant l'une des revendications 10 à 13, comprenant, en outre, un dispositif (21) d'étranglement monté dans le premier conduit (11) pour du gaz.

15. Système (10) de mesure suivant l'une des revendications 10 à 14, dans lequel la cellule (13) de mesure est une cellule (4) à trajet long ayant une marche (5) des rayons coudée plusieurs fois, qui a une section (6) d'absorption de 5 à 15 m, de préférence de 7 à 12 m.

16. Centrale (7) électrique ayant une turbine (8) à gaz, un générateur (9) de vapeur à récupération de la chaleur perdue et un système (10) de mesure suivant l'une des revendications 10 à 15.
